# EUROPEAN PATENT APPLICATION

(11) **EP 4 458 291 A1**
(43) Date of publication of application: **06.11.2024**
(21) Application number: 22914823.4
(22) Date of filing: 27.12.2022
(51) Int. Cl.: A61B 17/34, A61B 18/12, A61B 18/14

(54) **SLEEVE, INJECTION DEVICE, INJECTION SYSTEM, ABLATION DEVICE AND ABLATION SYSTEM**

(30) Priority: 31.12.2021 CN 202111679034
(71) Applicant: Hangzhou Deke Medtech Co., Ltd., Hangzhou, Zhejiang 310052 (CN)
(72) Inventor: SHAO, Nan, Hangzhou Zhejiang 310052 (CN); LI, Yang, Hangzhou Zhejiang 310052 (CN); ZHUANG, Zhenping, Hangzhou Zhejiang 310052 (CN); HU, Fanbo, Hangzhou Zhejiang 310052 (CN)
(74) Representative: Zaboliene, Reda
(86) International application number: PCT/CN2022/142505
(87) International publication number: WO 2023/125571

(57) **Abstract**

A sleeve (10), an injection device (200), an injection system (300), an ablation device (600), and an ablation system (700). The sleeve (10) includes a main body (11), the main body (11) defines a first channel (12) passing through the main body (11) in an axial direction, and a side wall of the main body (11) defines at least one second channel (13) which is communicated with the first channel (12). When the sleeve (10) abuts against the ventricular tissue wall, the fluid can only flow out from the second channel (13) in the side wall of the sleeve (10), which means that the needle can be advanced for treatment of the ventricular tissue.

## Description

### TECHNICAL FIELD

The present disclosure relates to the technical field of medical devices, and in particular to a sleeve, an injection device, an injection system, an ablation device and an ablation system.

### DESCRIPTION OF THE PRIOR ART

In the treatment of some heart diseases, such as myocardial obstruction, drugs need to be injected into the endocardium. During specific treatment, the guiding sheath for providing an interventional passage is introduced into the left ventricle through puncture via the femoral artery. The imaging system performs three-dimensional mapping of the heart tissue and obtains relevant images. The operator can judge whether the needle outlet contacts the target puncture point based on the images and determine whether to advance the needle. When the needle is advanced, the injection needle punctures the endocardium and injects drugs into the myocardial tissue.

However, due to the anatomical configuration of the heart tissue, there may be errors in the images generated by the imaging system. The operator cannot quickly judge from the images whether the needle outlet is in contact with the puncture point. Therefore, the operator needs to spend time to observe the images carefully before advancing the needle to determine whether to advance the needle, resulting in a time-consuming surgery.

### SUMMARY OF THE DISCLOSURE

In order to solve at least one of the above-mentioned problems existing in the prior art, in one aspect of the present disclosure, a sleeve is provided. The sleeve includes a main body, wherein the main body defines a first channel passing through the main body in an axial direction, and a side wall of the main body defines at least one second channel which is communicated with the first channel.

By providing a first channel in the axial direction in the main body and at least one second channel communicating with the first channel in the side wall, when injecting fluid into the proximal end of the first channel, the fluid can flow out through both the first channel and the second channel; but when the distal end surface of the main body abuts against the tissue wall, the fluid can only flow out from the second channel because the distal end of the first channel will be blocked. Therefore, it can be determined whether the distal end surface of the main body is in contact with the tissue wall of the myocardial tissue from the position of the fluid outflow. If the fluid only flows out from the second channel, then it can be determined that the distal end surface of the main body is in close contact with the tissue wall of the myocardial tissue, so the needle can be advanced for treatment. If the fluid flows out from the first and second channels simultaneously, it can be determined that the distal end surface of the main body is in partial contact or not in contact with the tissue wall of the myocardial tissue, so it is necessary to adjust the position of the sleeve before advancing the needle for treatment.

In the following, several alternatives are provided, but merely as further additions or preferences, instead of as additional limitations to the above-mentioned technical solution. Without technical or logical contradiction, the alternatives can be combined with the above-mentioned technical solution, individually or in combination.

Optionally, the main body includes a distal section, and the distal section is provided with an abutment surface for abutting a myocardial tissue.

Optionally, the sleeve further includes at least one sliding member, at least a part of the sliding member protrudes from the abutment surface, and the sliding member is configured to selectively open or close the second channel.

Optionally, the sliding member has a first position for opening the second channel and a second position for closing the second channel, and the sleeve further includes at least one spring element acting on the sliding member to move the sliding member toward the second position.

Optionally, the sleeve further includes at least one third channel communicated with the second channel, and the sliding member and the spring element are arranged in the third channel.

Optionally, a distal end surface of the main body is the abutment surface, a distal end of the third channel defines an opening on the abutment surface, and a proximal end of the third channel is closed or provided with a blocking member, wherein a distal end of the sliding member protrudes from the abutment surface through the opening, and the sliding member is configured to selectively open or close the second channel according to a position of the sliding member in the third channel.

Optionally, a proximal end of the sliding member is connected to the spring element, and a proximal end of the spring element abuts a proximal end of the third channel.

Optionally, the sliding member defines a through hole, and when the spring element is in a first state, the sliding member is in the first position, and the through hole communicates with the second channel.

When the spring element is in a second state, the sliding member is in the second position, and the sliding member blocks the second channel.

Optionally, an outer wall of the main body has a balance opening communicated to a proximal end of the third channel.

Optionally, the main body includes a proximal section, an outer diameter of the proximal section gradually increases from a proximal end to a distal end, and a radial dimension of a part of the first channel corresponding to the proximal section is a constant value or gradually decreases.

Optionally, the main body further includes a middle section extending from the proximal section to the distal section, the middle section defines the second channel, and a radial dimension of a part of the middle section adjacent to the proximal section is greater than a radial dimension of a part of the middle section adjacent to the distal section.

Optionally, a radial dimension of the distal section is a constant value, or a radial dimension of a part of the distal section adjacent to the middle section is greater than a radial dimension of a part of the distal section away from the middle section.

Optionally, at least one electrode is provided on a side wall of the main body, or the main body is made of metal.

The present disclosure further discloses an injection device, including:
a delivery assembly, including a catheter and the sleeve as mentioned above, a proximal end of the sleeve being connected to a distal end of the catheter; and
an injection assembly, which is movably provided in the catheter.

Optionally, the injection assembly includes a needle body, a needle connector and a needle connection tube, the needle connector is connected between the needle body and the needle connection tube, and the needle connection tube includes an inner tube and an outer tube.

A proximal end of the needle connector is arranged around the inner tube, the outer tube is arranged around a distal end of the needle connector, and the distal end of the needle connector is arranged around the needle body.

A side wall of the needle connector defines a communication hole, and a gap between the inner tube and the outer tube is communicated to an interior of the inner tube through the communication hole.

Optionally, at least one electrode is provided on a side wall of the main body, or the main body is made of metal.

A conductive wire is arranged in a wall of the catheter, a distal end of the conductive wire is connected to the electrode or the main body, and a proximal end of the conductive wire is connected to a mapping system.

The present disclosure further discloses an injection system, including a handle and an injection device as mentioned above. The handle is connected to a proximal end of the catheter, the handle is provided with at least one injection part, and the injection part, the catheter and the injection assembly are communicated with each other.

Optionally, the injection part includes a first injection part and a second injection part, and the first injection part and the second injection part correspond to different injection channels in the catheter and/or the handle.

The present disclosure further discloses an ablation device, including a delivery assembly and an ablation assembly, a distal end of the delivery assembly being provided with the sleeve as mentioned above; wherein the ablation assembly is movably inserted in the delivery assembly and includes an ablation needle, the delivery assembly is configured to be introduced into a heart through a catheter, and the ablation needle is configured to pass through the sleeve from the delivery assembly to puncture an endocardium and enter a myocardial tissue to ablate the myocardial tissue.

Optionally, the delivery assembly includes a guiding sheath and a bendable sheath, the bendable sheath is movably inserted in the guiding sheath, the ablation needle is movably inserted in the bendable sheath, and the sleeve is disposed at a distal end of the bendable sheath.

Optionally, at least part of the ablation needle is provided with an ablation section.

The present disclosure further discloses an ablation system, including: the ablation device as mentioned above; and an ablation energy generating device connected to the ablation device and configured to provide energy to the ablation device.

Optionally, the ablation system further includes a perfusion device for providing fluid to the ablation device.

### BRIEF DESCRIPTION OF DRAWINGS

FIG. 1 is a schematic view of the heart;
FIG. 2 is a schematic structural view of a sleeve according to an embodiment of the present disclosure;
FIG. 3 is a cross-sectional view of the sleeve in FIG. 2;
FIG. 4 is a schematic structural view of a sleeve according to another embodiment of the present disclosure;
FIG. 5 is a cross-sectional view of the sleeve in FIG. 4;
FIG. 6 is a view of the sleeve in FIG. 4 in one working state;
FIG. 7 is a view of the sleeve in FIG. 4 in another working state;
FIG. 8 is a schematic structural view of a catheter and a sleeve of an injection device according to an embodiment of the present disclosure;
FIG. 9 is a schematic structural view of an injection device according to an embodiment of the present disclosure;
FIG. 10 is an exploded view of an injection assembly shown in FIG. 9;
FIG. 11 is a schematic structural view of the catheter shown in FIG. 8;
FIG. 12 is a schematic view of the exposed part of the conductive wire in the catheter in FIG. 11;
FIG. 13 is a schematic view of a variant of the catheter in FIG. 8;
FIG. 14 is a schematic structural view of an injection system according to an embodiment of the present disclosure;
FIG. 15 is a schematic structural view of the handle shown in FIG. 14;
FIGS. 16 to 24 are schematic views showing the process of cardiac tissue wall injection using the injection system of the present disclosure;
FIG. 25 is a schematic structural view of an ablation assembly of an ablation device according to an embodiment of the present disclosure; and
FIG. 26 is a schematic structural view of an ablation system according to an embodiment of the present disclosure.

### DESCRIPTION OF EMBODIMENTS

First of all, it should be noted that in the field of interventional medical device, the proximal end refers to the end close to the operator, and the distal end refers to the end far away from the operator. The axial direction refers to the direction parallel to the line connecting the distal center and the proximal center of the medical device, the radial direction refers to the direction perpendicular to the axial direction, and the circumferential direction refers to the direction surrounding the axial direction.

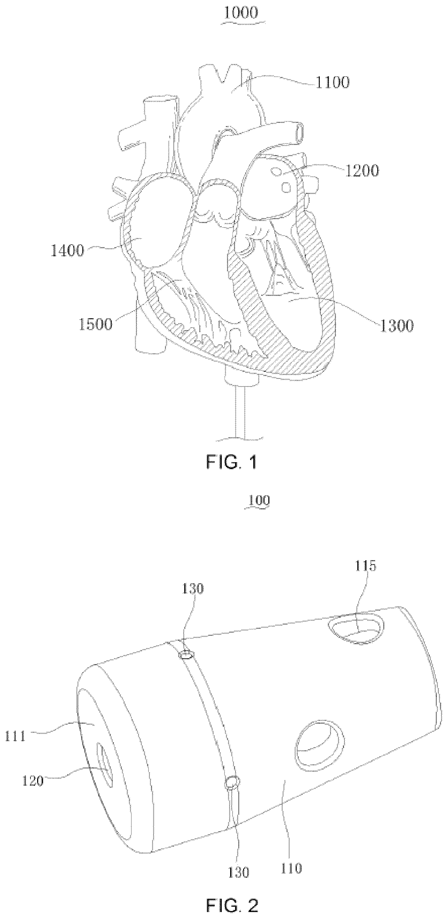

FIG. 1 is a schematic view of a heart 1000, which mainly includes an aortic arch 1100, a left atrium 1200, a left ventricle 1300, a right atrium 1400 and a right ventricle 1500.

Referring to FIGS. 2 to 3, an embodiment of the present disclosure discloses a sleeve 100, which can be used with an interventional medical device to treat various diseases of the heart 1000, for example, to inject hydrogel for treatment of myocardial obstruction, or for ablation in hypertrophic cardiomyopathy or the like. With the three-dimensional mapping system, it is possible to determine whether the sleeve 100 is in contact with the tissue wall by injecting fluid into the sleeve 100 in this embodiment and based on the fluid outflow, and accordingly determine whether to advance the needle.

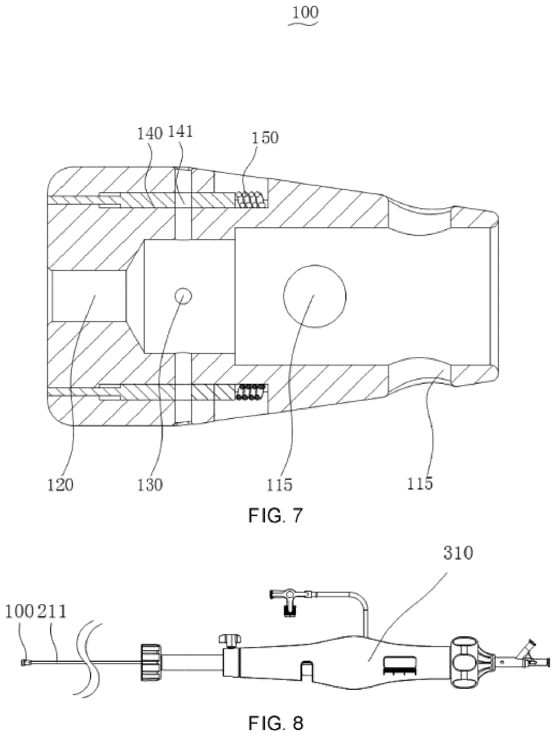

Referring to FIGS. 8 to 10, in use, the sleeve 100 in this embodiment is connected to the catheter 211, and introduced into the left ventricle 1300 through the catheter 211 via the aortic arch 1100. When the needle can be advanced for treatment, the needle body 221 is pushed through the catheter 211 and the sleeve 100 to puncture the endocardium to treat the myocardial tissue.

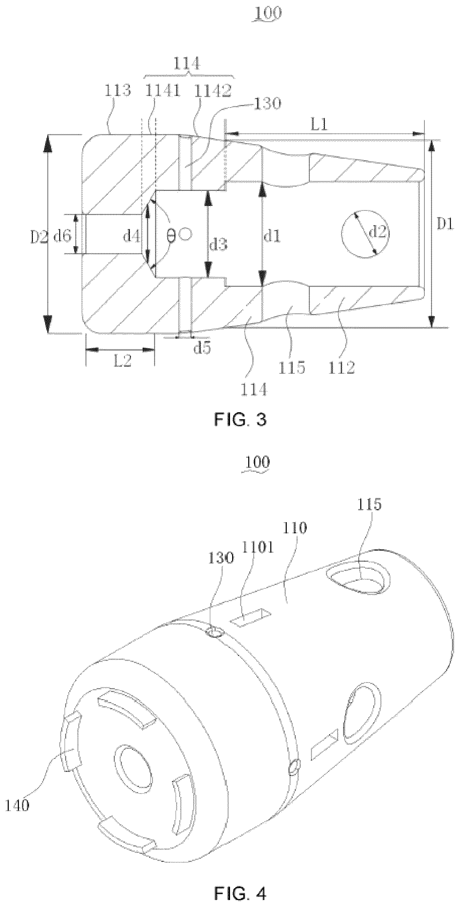

Referring to FIGS. 2 and 3, a sleeve 100 according to a first embodiment of the present disclosure includes a main body 110. The main body 110 defines a first channel 120 that passes through the main body 110 in the axial direction, the side wall of the main body 110 defines at least one second channel 130, and the first channel 120 is communicated with the at least one second channel 130.

Specifically, based on the above arrangement, after injecting fluid into the proximal end of the sleeve 100, fluid can flow out through both the first channel 120 and the second channel 130. When the distal end of the first channel 120 is blocked, fluid can only flow out through the second channel 130. Therefore, when the distal end surface of the main body 110 abuts against the tissue wall of the myocardial tissue, the distal end of the first channel 120 is blocked, and fluid can only flow out through the second channel 130. Therefore, when the sleeve 100 is arranged around the end of the injection assembly or the ablation assembly, it can be determined if the distal surface of the main body 110 is in close contact, or in partial contact, or not in contact with the tissue wall of the myocardium tissue by infusing fluid into the proximal end of the sleeve 100, depending on whether fluid flows out of the first channel 120. If it is determined that the distal surface of the main body 110 is in close contact with the tissue wall of the myocardium tissue, the needle can be advanced for treatment. Otherwise, the distal surface of the sleeve 100 needs to be adjusted.

The sleeve 100 in this embodiment can be made of metal or non-metal material. The metal material can be, for example, 304 stainless steel, 316 stainless steel, nickel-titanium alloy, etc., and the non-metal material can be, for example, polycarbonate (PC). In a specific embodiment of the present disclosure, the sleeve 100 is preferably made of 316 stainless steel.

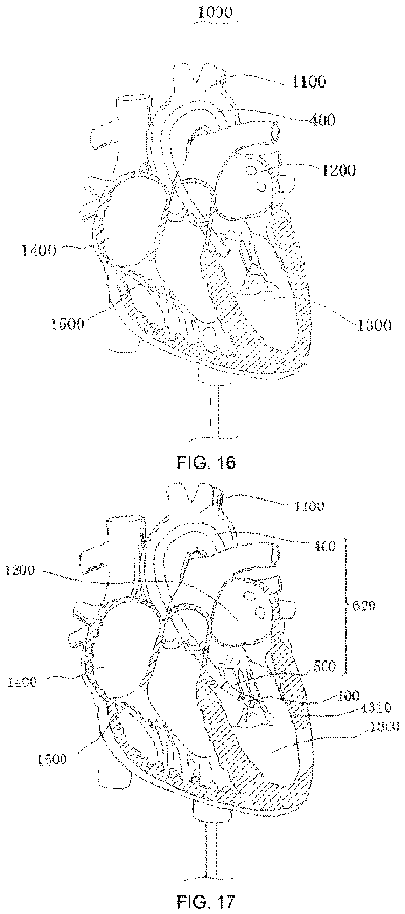

Referring to FIGS. 2 and 17, in this embodiment, the distal section of the main body 110 has an abutment surface 111 for abutting against the tissue wall 1310 of the myocardial tissue. Specifically, in order to ensure that the abutment surface 111 can closely contact the tissue wall 1310 of the myocardial tissue, the abutment surface 111 in this embodiment is a smooth plane. After the smooth abutment surface 111 abuts against the tissue wall 1310, it can be adjusted to closely contact the tissue wall 1310 of the myocardial tissue under the action of external force, and damage to the tissue wall can also be avoided.

Referring to FIG. 3, in some embodiments, the main body 110 includes a proximal section 112, a middle section 114, and a distal section 113 connected in sequence. The parts of the first channel 120 corresponding to the proximal section 112, the middle section 114, and the distal section 113 have different radial dimensions, so that fluid can be converged from the position with a large radial dimension to the position with a small radial dimension, to facilitate outflow through the first channel 120 or the second channel 130.

Referring to FIGS. 2 and 8, in this embodiment, the proximal section 112 is used to connect to the catheter 211. When connecting the proximal section 112 and the catheter 211, the catheter 211 can be arranged around the proximal section 112, so that the outer wall of the proximal section 112 is connected to the inner wall of the catheter 211, or the proximal section 112 can be arranged around the catheter 211 so that the outer wall of the catheter 211 is connected to the inner wall of the proximal section 112.

Referring to FIG. 3, in the case where the proximal section 112 is arranged around the catheter 211 and the outer wall of the catheter 211 is connected to the inner wall of the proximal section 112, the outer diameter D1 of the proximal section 112 gradually increases from the proximal end to the distal end and the radial dimension d1 of the part of the first channel 120 corresponding to the proximal section 112 is a constant value or gradually decreases. In the case where the proximal section 112 surrounds the distal end of the catheter 211, by gradually increasing the outer diameter D1 of the proximal section 112 from the proximal end to the distal end, the height of the transition step between the sleeve 100 with the gradually decreasing outer diameter and the catheters 211 is reduced, so the sleeve 100 can transition to the catheters 211 as smooth as possible, preventing blood flow from impacting the step surface to form thrombus during the surgery, and reducing damage to the human body.

In other embodiments, the outer diameter D1 of the proximal section 112 can be constant, so that the outer diameter of the entire sleeve 100 is consistent, facilitating the manufacture of the sleeve, without additional setting of the outer diameter of this section, reducing manufacture difficulty. In this case, when connecting the sleeve 100 with the catheter 211, the catheter 211 can be arranged around the proximal section 112.

In the case where the radial dimension d1 of the part of the first channel 120 corresponding to the proximal section 112 is a constant value, the proximal section 112 can be conveniently connected to the catheter 211 when arranging the proximal section 112 around the outer wall of the distal end of the catheter 211 which has a constant outer diameter. In the case where the radial dimension d1 of the part of the first channel 120 corresponding to the proximal section 112 gradually decreases, the proximal section 112 and the catheter 211 have an interference fit when arranging the proximal section 112 around the outer wall of the distal end of the catheter 211 for connection, increasing the connection strength between the two.

Specifically, in this embodiment, the catheter 211 is connected with the proximal section 112 corresponding to a constant radial dimension d1. In this case, an assembly clearance t for filling glue is reserved between the side wall of the part of the first channel 120 corresponding to the proximal section 112 and the outer wall of the catheter 211, so that the proximal section 112 and the outer wall of the catheter 211 can be bonded together. The assembly clearance t ranges from 0.1 mm to 0.2 mm, and the radial dimension d1 ranges from 2.0 mm to 3.0 mm.

In some embodiments, the assembly clearance t = 0.125 mm and the radial dimension d1 = 2.65 mm, to adapt to the size of the catheter 211, facilitating the assembly of the catheter 211, and allowing an appropriate amount of glue to be filled.

In the case where the side wall of the first channel 120 and the outer wall of the catheter 211 are bonded by glue, in order to facilitate the connection between the two, a glue injection hole 115 is opened in the proximal section 112 in this embodiment, so that glue can be injected into the clearance between the sleeve 100 and the catheter 211 through the glue injection hole 115. The quantity of the glue injection hole 115 defined on the proximal section 112 can be one or more, and the glue injection hole 115 can be a circular hole, a square hole, or other polygonal holes.

Specifically, a plurality of glue injection holes 115 are opened in the side wall of the proximal section 112 in this embodiment, so that glue can be injected from the plurality of glue injection holes 115, improving the assembly efficiency of the sleeve 100 and the catheter 211. Further, through the plurality of glue injection holes 115, glue can flow into a more uniform distribution, ensuring the connection strength. In this embodiment, four glue injection holes 115 are provided, which are arranged in two pairs opposite to each other in the proximal section 112, and the two pairs of glue injection holes 115 are offset from each other, so that the glue can flow throughout the assembly clearance t, to improve the tightness of the connection between the sleeve 100 and the catheter 211.

Specifically, the glue injection hole 115 in this embodiment is a circular hole, to facilitate processing. The diameter d2 of the glue injection hole 115 is 1.2 mm, facilitating the injection of glue. The length L1 of the proximal section 112 ranges from 3.0 mm to 5.0 mm, ensuring a sufficient connection length with the catheter 211, thereby ensuring the stability of the connection. Preferably, the length L1 of the proximal section 112 in this embodiment is 5.0 mm.

The transition section between the proximal section 112 to the distal section 113 is the middle section 114. The middle section 114 is provided with a second channel 130. The corresponding radial dimension d3 of the part of the middle section 114 close to the proximal section 112 is greater than the corresponding radial dimension d4 of the part of the middle section 114 close to the distal section 113. The proximal section 112 is configured to connect with the catheter 211, and fluid can flow through the interior lumen of the catheter 211 and into the sleeve 100. When fluid flows from the part of the first channel 120 corresponding to the proximal section 112 into the part of the first channel 120 corresponding to the distal section 113, the channel with a reduced inner diameter can converge the fluid.

Specifically, the middle section 114 in this embodiment includes two parts corresponding to the first channel 120: a first part 1141 close to the proximal section 112 and a second part 1142 close to the distal section. The radial dimension of the first channel 120 corresponding to the first part 1141 is d3, the radial dimension of the first channel 120 corresponding to the second part 1142 is d4, and the radial dimension d3 is greater than the radial dimension d4, so that the fluid flowing from the proximal section 112 to the distal section 113 can be converged.

In some embodiments, radial dimension d1 > radial dimension d3 > radial dimension d4, so that the size of the part of the first channel 120 corresponding to the middle section changes from large to small, and fluid gradually flows toward the part of the first channel 120 with a smaller radial dimension and flows out from the first channel 120 or the second channel 130 after being converged.

The radial dimension d3 in this embodiment ranges from 1.8 mm to 2.5 mm. After assembly with the catheter 211, the radial dimension d3 > the inner diameter of the catheter 211. In other embodiments, after assembly with the catheter 211, the radial dimension d2 can be less than or equal to the inner diameter of the catheter 211. In a specific embodiment of the disclosure, d3 = 2.2 mm.

The radial dimension d4 in this embodiment gradually decreases toward the distal section 113, so that the side wall of the part of the first channel 120 corresponding to the second part 1142 is tapered, so as to converge the fluid flowing out from the catheter 211 into the part of the first channel 120 corresponding to the distal section 113 through the second part 1142.

The cross-sectional shape of the second channel 130 in this embodiment can be circular, square or other geometric shapes. In this embodiment, the cross-sectional shape of the second channel 130 is circular, facilitating processing and manufacture. The radial dimension d5 of the second channel 130 ranges from 0.1 mm to 0.5 mm. In one embodiment of the present disclosure, d5 = 0.3 mm, ensuring that fluid can flow out of the second channel 130 smoothly.

In this embodiment, the radial dimension d6 corresponding to the distal section 113 is a constant value, or the radial dimension corresponding to the part of the distal section 113 close to the middle section 114 is greater than the radial dimension corresponding to the part of the distal section 113 away from the middle section 114 so as to converge the fluid. In the case where the radial dimension corresponding to the part of the distal section 113 close to the middle section 114 is greater than the radial dimension corresponding to the part of the distal section 113 away from the middle section 114, the corresponding radial dimension d6 of the distal section 113 can be graded, that is, the part of the first channel 120 corresponding to the distal section 113 can include multiple sections. It should be noted that the radial dimension d6 of the part of the first channel 120 corresponding to the distal section 113 should be large enough to ensure the free movement of the needle body when the needle is pushed out from the part of the first channel 120 corresponding to the distal section 113.

In some embodiments, the radial dimension d6 corresponding to the distal section 113 is a constant value, facilitating the processing and manufacture of the sleeve 100. The radial dimension d6 of the first channel 120 corresponding to the distal section 113 in this embodiment is greater than the outer diameter of the needle body, and the radial dimension d6 ranges from 0.5 mm to 1.5 mm, and more preferably d6 = 1.0 mm.

The corresponding outer diameter of the distal section 113 is D2. The larger the outer diameter D2, the larger the area of the abutment surface 111 and the larger the contact area with the target tissue wall 1310, and thus the more stable the abutment will be. However, it should be noted that as the outer diameter D2 increases, the flexibility decreases, making the operation inconvenient. The outer diameter D2 in this embodiment ranges from 3.0 mm to 6.0 mm. In a specific embodiment of the present disclosure, the outer diameter D2 = 5.0 mm.

In some embodiments, the cone angle of the part of the first channel 120 corresponding to the second part 1142 of the middle section 114 is θ, and the sum of the lengths of the distal section 113 and the second part 1142 of the sleeve 100 is L2. The cone angle θ is inversely proportional to the length L2. The larger θ is, the shorter L2 is. The smaller θ is, the longer L2 is. Further, the length L2 is inversely proportional to the flexibility of the catheter 211. The longer L2 is, the lower the flexibility of the catheter 211 is. The shorter L2 is, the higher the flexibility of the catheter 211 is. Accordingly, the θ in this embodiment ranges from 90° to 140°, and L2 ranges from 1.5 mm to 2.0 mm. In a specific embodiment of the present disclosure, θ = 120°, and L2 = 1.85 mm.

Referring to FIGS. 4 to 7, in some embodiments, another sleeve 100 is provided. The differences between the sleeve 100 in this embodiment and the sleeve 100 in the above embodiment are as follow.

The sleeve 100 in this embodiment further includes at least one sliding member 140, at least one spring element 150, and at least one third channel 160.

The third channel 160 is communicated to the second channel 130, and the distal surface of the main body 110 is the abutment surface 111. The distal end of the third channel 160 has an opening 161 on the abutment surface 111, and the proximal end of the third channel 160 is closed or provided with a blocking member. The sliding member 140 and the spring element 150 are arranged in the third channel 160. The distal end of the sliding member 140 protrudes from the abutment surface 111 through the opening 161, and the proximal end of the sliding member 140 is connected to the spring element 150. The proximal end of the spring element 150 abuts the proximal end of the third channel 160. Since the sleeve 100 is provided with the sliding member 140, the spring element 150 and the third channel 160, the second channel 130 can be closed or opened by the sliding member 140. After fluid is injected into the proximal end of the sleeve 100, if the abutment surface 111 of the sleeve 100 is in partial contact or not in contact with the tissue wall, the first channel 120 is opened but the second channel 130 is not opened, and fluid will only flow out from the first channel 120; if the abutment surface 111 of the sleeve 100 is in close contact with the tissue wall, the first channel 120 is not opened and the second channel 130 is opened, and fluid will only flow out from the second channel 130.

The outer wall of the main body 110 has a balance opening 1101 communicated with the proximal side of the third channel 160. When the sliding member 140 moves, the build-up fluid pressure on the proximal side of the third channel 160 can be relieved through the balance opening, to prevent the movement of the sliding member 140 from being resisted, maintaining pressure balance. In order to facilitate the assembly of the spring element 150, the main body 110 can be formed by separate pieces that are mutually snap-fastenable in the axial or radial direction.

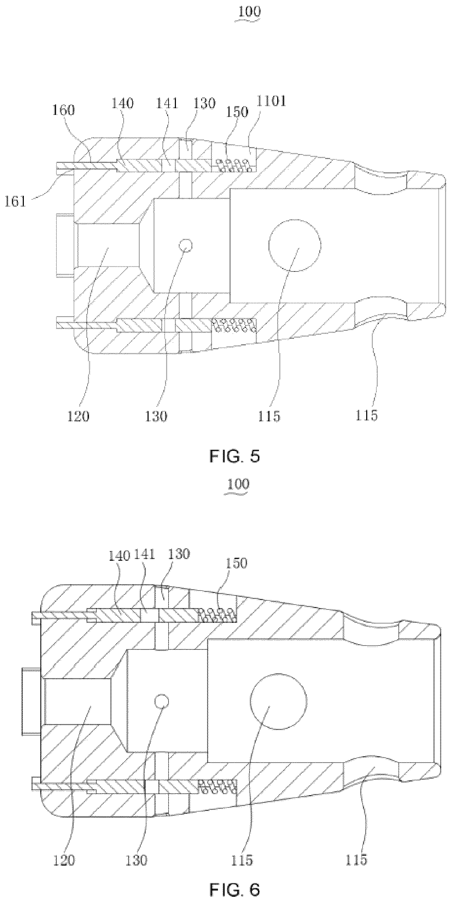

Specifically, referring to FIG. 7, the sliding member 140 defines a through hole 141. When the spring element 150 is in a first state, the sliding member 140 is in a first position, and the through hole 141 is communicated with the second channel 130. Referring to FIG. 5, when the spring element 150 is in a second state, the sliding member 140 is in a second position, and the sliding member 140 blocks the second channel 130. It can be understood that, in this embodiment, when the distal end of the sliding member 140 protrudes from the abutment surface 111 through the opening 161, the spring element 150 is in the second state, that is, in the natural state, the sliding member 140 blocks the second channel 130, fluid can only flow out from the first channel 120, and thus it can be determined that the abutment surface 111 of the sleeve 100 is not in contact with the tissue wall 1310. When the sliding member 140 compresses the spring element 150 so that the spring element 150 is in the first state, the sliding member 140 is in the first position, and the through hole 141 is communicated with the second channel 130, fluid can only flow out from the second channel 130 and the through hole 141, and thus it can be determined that the abutment surface 111 of the sleeve 100 is in close contact with the tissue wall 1310.

In addition, compared with the sleeve 100 mentioned above, in use of the sleeve 100 provided with the sliding member 140, the spring element 150 and the third channel 160 in this embodiment, when the abutment surface 111 of the sleeve 100 is in partial contact with the tissue wall 1310, the through hole 141 in the sliding member 140 can partially overlap with the second channel 130 so that both the first channel 120 and the second channel 130 are opened (see FIG. 6). As the second channel 130 is narrowed, the outflow of fluid per unit time can be reduced, so the consumption of the contrast medium can be reduced, especially in the case of multiple injection locations.

At least one sliding member 140 can be provided. It can be understood that since the abutment surface 111 of the sleeve 100 needs to abut against the tissue wall 1310, the abutment surface 111 has a certain area. In order to make the determination of whether the abutment surface1 11 is in close contact with the tissue wall more accurately, in this embodiment, it is preferred to provide four sliding members 140, and the four sliding members 140 are evenly arranged on the sleeve 100.

Since the sleeve 100 needs to transmit electrical signals to the mapping system, at least one electrode can be provided on the side wall of the main body 110 in this embodiment. The electrode is coupled to the mapping system through a conductive wire, and the mapping system applies excitation signals of different frequencies to the electrode located on the sleeve 100 through the conductive wire, and captures the electric field signals or magnetic field signals generated by the electrode to achieve the purpose of three-dimensional mapping, thereby observing the condition of the sleeve 100.

In another embodiment, the sleeve main body 110 can be made of metal material, so that the main body 110 can be directly coupled to the conductive wire. That is, the electrical signal can be transmitted to the main body 110 through the mapping system.

When the sleeve 100 above is used for treatment of the heart 1000, the guiding sheath 400 for providing an interventional passage is introduced into the left ventricle 1300 through puncture via the femoral artery. The catheter 211 pushes the sleeve 100 through the interior lumen of the guiding sheath 400 into the left ventricle 1300. When fluid is injected into the catheter 211, the fluid flows into the sleeve 100. Based on the outflow of the fluid in the first channel 120 and/or the second channel 130, it can be determined whether the sleeve 100 is in close contact with the tissue wall 1310, and accordingly it can be determined whether to advance the needle. If the fluid only flows out from the second channel 130, it can be determined that the abutment surface 111 is in close contact with the tissue wall 1310, and the first channel 120 is blocked, so the needle can be advanced for treatment. Otherwise, further adjustment is needed.

Referring to FIGS. 8 to 13, in some embodiments, an injection device 200 is disclosed, including a delivery assembly 210 and an injection assembly 220.

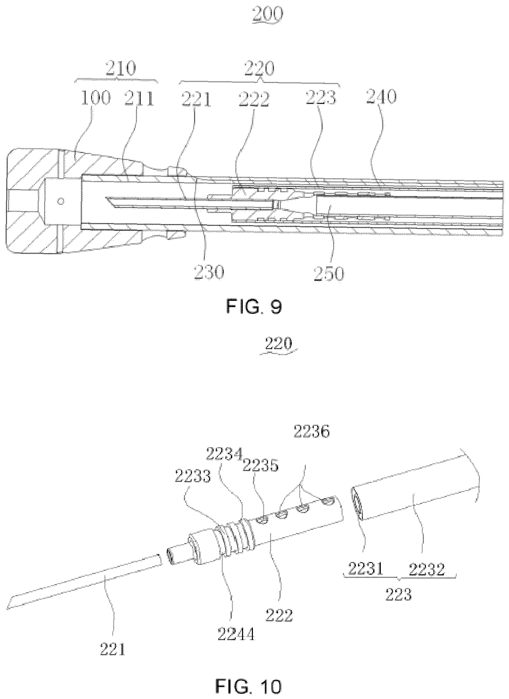

Referring to FIGS. 8 and 9, the delivery assembly 210 includes a catheter 211 and the sleeve 100 mentioned above. The proximal end of the sleeve 100 is connected to the distal end of the catheter 211. The injection assembly 220 is movably received in the catheter 211, for injecting liquid medicine into the lesion tissue.

The above-mentioned injection device 200 is also provided with the sleeve 100 at the distal end of the catheter 211. The sleeve 100 can be the sleeve according to any of the above embodiments. When injecting fluid into the sleeve 100, depending on the outflow of the fluid in the first channel 120 or the second channel 130 in the sleeve 100, it can be determined whether the sleeve 100 is in close contact with the tissue wall 1310 and whether the injection assembly 220 can advance the needle to inject the liquid medicine for treatment of the myocardial tissue. As long as the fluid does not flow out from the first channel 120, it can be determined that the abutment surface 111 is in contact with the tissue wall 1310, the first channel 120 is blocked by the tissue wall, and the needle can be advanced for treatment.

In the surgery, it is necessary to input different fluids into the sleeve 100 in order. For example, the contrast fluid is first injected for injection location selection, and then the liquid medicine is injected through the injection assembly 220. In order to facilitate the operation, the catheter 211 and the injection assembly 220 in this embodiment define different channels to inject different fluids.

Specifically, referring to FIG. 9, a gap is defined between the catheter 211 and the injection assembly 220. The gap is configured as a first fluid channel 240. The injection assembly 220 defines a second fluid channel 250 therein. Fluid can flow into the first fluid channel 240 and the second fluid channel 250 respectively. For example, a contrast medium can be injected through the first fluid channel 240 for injection location selection, and then a contrast medium can be injected through the second fluid channel 250 to determine whether the treatment conditions are met. When it is determined that the treatment conditions are met, the liquid medicine can be injected through the second fluid channel 250 for treatment of the myocardial tissue.

It can be understood that since the mapping system needs to apply electrical signals to the sleeve 100, the mapping system needs to be electrically coupled to the sleeve 100, so a conductive wire 230 is connected between the two. The distal end of the conductive wire 230 is connected to the electrode or the main body 110, and the proximal end of the conductive wire 230 is connected to the mapping system. Therefore, the signals from the sleeve 100 can be received by the mapping system through the conductive wire 230 connecting between the sleeve 100 and the mapping system.

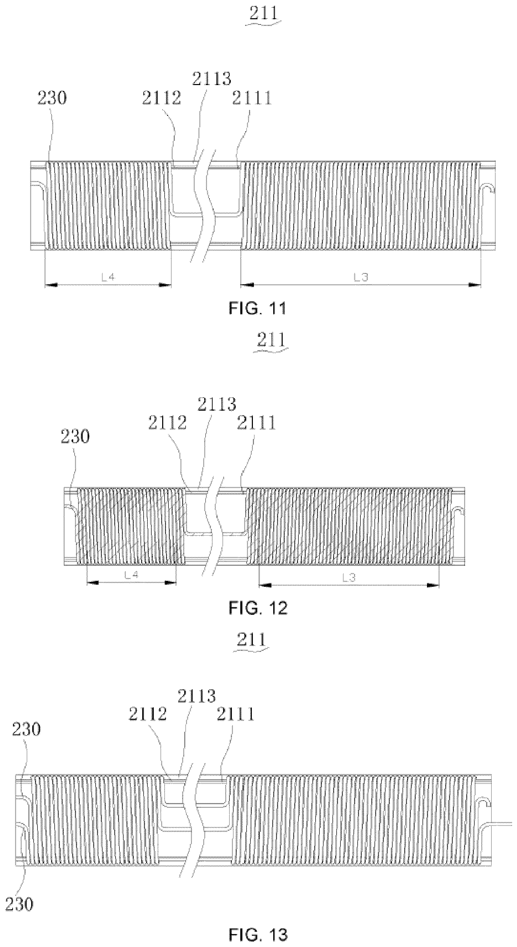

Referring to FIGS. 11 to 13, the conductive wire 230 in this embodiment is buried in the wall of the catheter 211. Specifically, the catheter 211 includes from the inside to the outside: an inner film 2111, a metal braided mesh layer 2112 surrounding the inner film 2111, and a polymer layer 2113 surrounding the metal braided mesh layer 2112. The conductive wire 230 is wound around the metal braided mesh layer 2112. The metal braided mesh layer 2112 is made of stainless steel, and the polymer layer 2113 can be made of PEBAX.

The conductive wire 230 can be made of metal wires such as gold wire, silver wire, copper wire, etc. Copper wire has small resistance, but has poor strength. Silver wire and gold wire have smaller resistance, but has high density and thus heavy weight. Therefore, copper wire is preferably used as the conductive wire in this embodiment.

Specifically, when arranging the copper wire in the catheter 211, the copper wire covers the outer surface of the stainless steel braided mesh layer. In order to prevent the copper wire from directly contacting the metal braided mesh layer 2112 to form electrical conduction, the surface of the copper wire is covered with an insulating material.

The distal end of the copper wire is connected to the sleeve 100, and the proximal end thereof is connected to the mapping system. In order to ensure the conductivity, in this embodiment, both the distal and proximal sections of the copper wire of the conductive wire 230 are wound into coils, and the outer insulation layer and the polymer layer 2113 of the distal and proximal sections of the catheter 211 are scraped off, to improve the conductivity.

Referring to FIG. 12, the catheter 211 needs to be bendable so that it can detect the injection location normally for surgery. For example, when the distal end of the catheter 211 together with the sleeve 100 is introduced into the heart, the catheter 211 needs to be bent so that the abutment surface 111 of the sleeve 100 can abut against the tissue wall 1310. The flexibility of the distal and proximal sections of the catheter 211 is inversely proportional to the number of wound coils of the conductive wire 230. The more wound coils of the conductive wire 230, the higher the hardness, the lower the flexibility of the section of the catheter 211 with the coils. The less wound coils of the conductive wire 230, the lower the hardness, the higher the flexibility of the section of the catheter 211 with the coils. Therefore, in this embodiment, the first length L3 of the exposed proximal section of the conductive wire 230 is preferably in the range of 4.0 mm to 8.0 mm, more preferably 6.0 mm. The first length L4 of the exposed distal section of the conductive wire 230 is preferably in the range of 1.0 mm to 2.0 mm, more preferably 1.5 mm.

The quantity of the conductive wire 230 in this embodiment can be one, two or more. In order to ensure mapping accuracy, the quantity of the conductive wire 230 is two in this embodiment (see FIG. 13). The outer diameter of the conductive wire 230 is preferably in the range of 0.05 mm to 0.2 mm, and more preferably 0.1 m. Since the larger the outer diameter of the wire, the larger the outer diameter of the catheter, resulting in reduced flexibility, bending resistance and bendability of the catheter, the outer diameter of the conductive wire 230 is in the range of 0.05 mm and 0.2 mm, ensuring the flexibility of the catheter 211.

Referring to FIGS. 9 and 10, the injection assembly 220 includes a needle body 221, a needle connector 222 and a needle connection tube 223. The needle connector 222 is used to connect the needle body 221 and the needle connection tube 223, so that the needle body 221 and the needle connection tube 223 can be connected in one piece. Therefore, the liquid medicine such as hydrogel can be supplied to the needle body 221 through the needle connection tube 223.

Specifically, referring to FIG. 9, the needle connection tube 223 includes an inner tube 2231 and an outer tube 2232. There is a gap between the inner tube 2231 and the outer tube 2232. The proximal end of the needle connector 222 is arranged around the inner tube 2231, the outer tube 2232 is arranged around the distal end of the needle connector 222, and the distal end of the needle connector 222 is arranged around the needle body 221, thereby connecting the three into one piece.

As can be seen in FIG. 10, the side wall of the needle connector 222 has a communication hole 2235. The fluid between the inner tube 2231 and the outer tube 2232 in the radial direction flows into the interior of the inner tube 2231 through the communication hole 2235 after reaching the distal end. It can be seen from the figure that in addition to the communication hole 2235, the side wall of the needle connector 222 has a connection hole 2236. Through the connection hole 2236, glue dispensing or spot welding can be performed to strengthen the connection of the needle connector 222 with the inner tube 2231.

In order to enhance the connection stability of the distal end of the needle connection tube 223 and the needle connector 222, the needle connection tube 223 is provided with a plurality of spaced protrusions 2233 on the outer wall of the distal section thereof. The needle connection tube 223 and the needle connector 222 are heat-fused together through the protrusions 2233, and the grooves 2234 between the protrusions 2233 are filled with materials after heat-fusing, thereby increasing the connection force between the needle connection tube 223 and the needle connector 222 and increasing the connection stability.

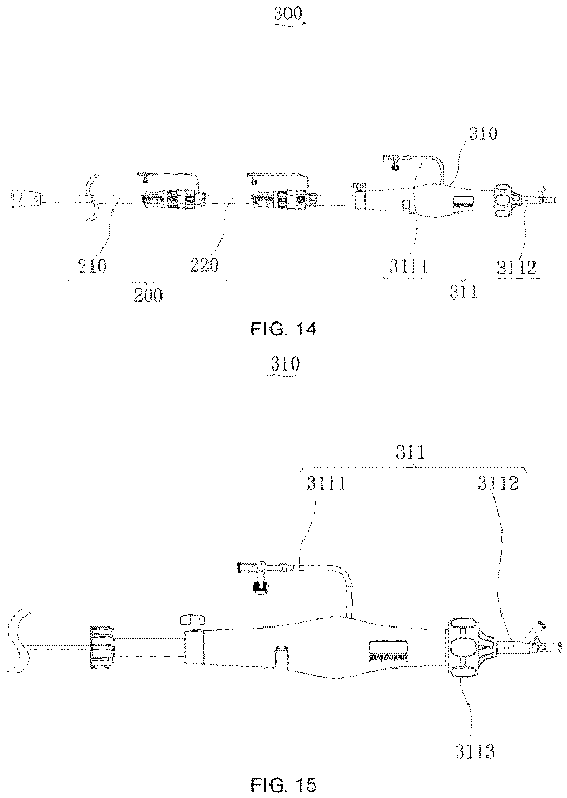

Referring to FIGS. 14 and 15, in some embodiments, an injection system 300 is disclosed, including a handle 310 and the above-mentioned injection device 200.

The handle 310 is connected to the proximal end of the catheter 211. The handle 310 is provided with at least one injection part 311. The injection part 311, the catheter 211, and the injection assembly 220 are communicated with each other, so that fluid can be injected through the injection part 311 of the handle 310, and flow through the catheter 211 and the sleeve 100 sequentially and then flow out through the first channel 120 and/or the second channel 130 of the sleeve 100, or fluid can flow through the injection device 200 via the injection part 311. Depending on the monitoring results of the mapping system, when the treatment conditions are met, the liquid medicine can be injected into the target tissue through the needle body 221 of the injection assembly 220.

Specifically, referring to FIGS. 14 and 15, in order to inject different fluids into the catheter 211 and the sleeve 100 for different surgical procedures, the injection part 311 in this embodiment includes a first injection part 3111 and a second injection part 3112. The first injection part 3111 and the second injection part 3112 correspond to different injection channels in the catheter 211 and/or the handle 310, so that different liquid medicines can be injected through the first injection part 3111 and the second injection part 3112 for surgery.

Specifically, for example, the first injection part 3111 is communicated to the first fluid channel 240, and the second injection part 3112 is provided in the axial direction of the handle 310 and communicated to the second fluid channel 250. During the treatment of myocardial obstruction, a contrast medium is first injected into the first injection part 3111, and it is determined whether to advance the needle according to the outflow direction of the contrast medium in the sleeve 100. After the needle is pushed out, a contrast medium is first injected into the second injection part 3112 to determine whether the sleeve 100 is in contact with the correct tissue wall, and if the sleeve 100 is positioned at the correct position, hydrogel or other liquid medicines can be injected through the second injection part 3112 and delivered to the target tissue. It can be seen from the figure that the second injection part 3112 has a three-way structure, with two fluid input ports at the proximal end, which can be communicated to the interior of the inner tube and the radial gap between the inner tube and the outer tube respectively, and accordingly contrast medium, hydrogel, or other liquid medicines can be switched as needed.

Referring to FIGS. 16 to 24, the method of using the injection system 300 in this embodiment for hydrogel injection includes the following steps.

In step S11, as shown in FIG. 16, a standard puncture via the femoral artery is performed, and the guiding sheath 400 is introduced into the human body via the femoral artery puncture and reaches the left ventricle 1300 of the heart 1000 through the aortic arch 1100.

In step S12, as shown in FIG. 17, the catheter 211 covered with the bendable sheath 500 is introduced into the human body along the guiding sheath 400 and reaches the left ventricle 1300.

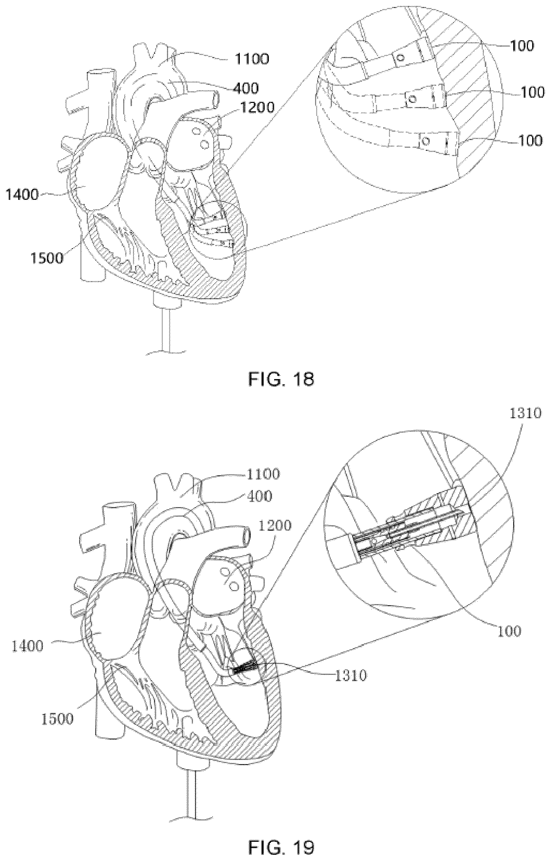

In step S13, the catheter 211 is electrically coupled to the mapping system, after the mapping system completes modeling, the catheter 211 is bent and moved with the assistance of the guiding sheath 400 and the bendable sheath 500, the location of the moving sleeve 100 is observed from the three-dimensional image transmitted from the sleeve 100, and the abutment surface 111 of the sleeve 100 is adjusted to vertically point to the tissue wall 1310 of the lesion tissue, as shown in FIG. 18.

In step S14, under the guidance of DSA (Digital subtraction angiography) and ultrasound images, the abutment surface 111 of the sleeve 100 is adjusted to abut against the tissue wall 1310 of the lesion tissue, as shown in FIG. 19.

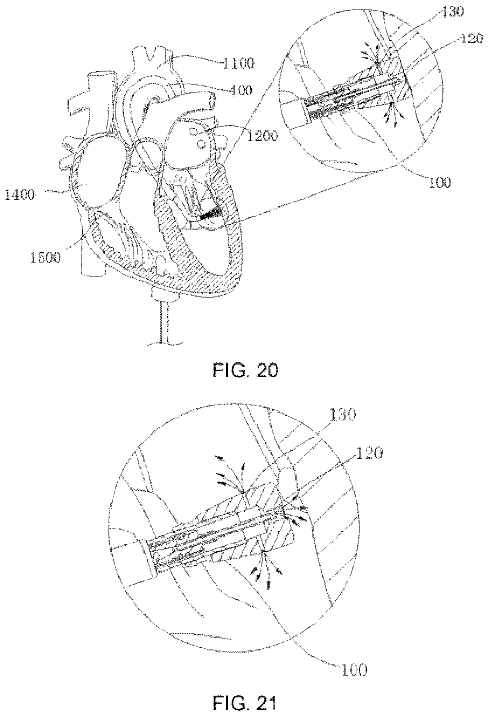

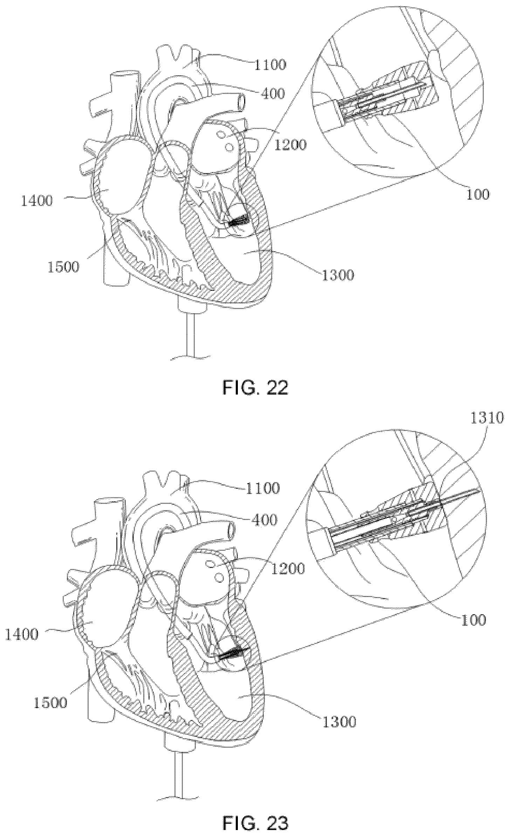

In step S15, a contrast medium is injected into the first injection part 3111 of the handle 310, so that the contrast medium flows through the catheter 211 and the sleeve 100 in sequence, and flows out from the first channel 120 and/or the second channel 130 of the sleeve. Now, the DSA image is used to determine whether the abutment surface 111 is in close contact with the tissue wall 1310. If the contrast medium flows out from the first channel 120 and the second channel 130 at the same time, as shown in FIG. 21, it means that the abutment surface 111 of the sleeve 100 is still not in contact with or only in partial contact with the tissue wall 1310 of the lesion tissue. FIG. 22 shows that the abutment surface 111 of the sleeve 100 is only in partial contact with the tissue wall 1310 of the lesion tissue. If the contrast medium only flows out from the second channel 130 of the sleeve 100, as shown in FIG. 20, it means that the abutment surface 111 of the sleeve 100 has closely contacted the tissue wall 1310, and the next step can be performed. Otherwise, steps S13 and S14 need to be repeated until the abutment surface 111 of the sleeve 100 is in close contact with the tissue wall 1310.

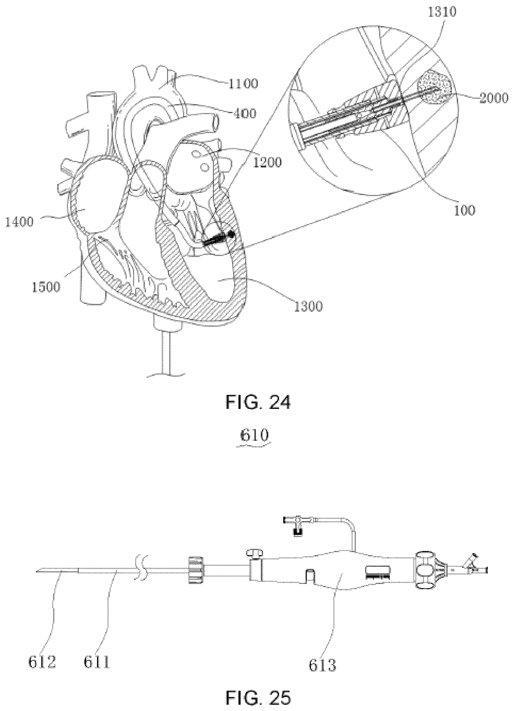

In step S16, as shown in FIGS. 23 and 24, the needle body 221 is driven by the driving member 3113 of the handle 310 to penetrate into the tissue wall 1310. A contrast medium is first injected into the second injection part 3112 of the handle 310. By observing the DSA image, if the contrast medium spreads or flows in a certain direction, it means that the needle body 221 may have penetrated into a groove of a pectinate muscle or into a blood vessel. In this case, the conditions for injecting hydrogel are not met, and the needle needs to be withdrawn and the previous steps are repeated to find the correct tissue wall 1310 and align the abutment surface 111 of the sleeve 100 with the correct tissue wall 1310 until the following conditions are met: under the DSA image, the contrast medium forms a clump and beats with the heart. In this case, the hydrogel 2000 can be injected through the second injection part 3112 into the lesion tissue. The needle body 221 is withdrawn after the injection is completed.

In step S17, the above steps are repeated to find the second injection point, and so on. After injections at all desired injection points, the guiding sheath 400, catheter 211 and sleeve 100 are withdrawn, thereby finishing the injection surgery.

Referring to FIGS. 17 and 25, in some embodiments, an ablation device is disclosed, including a delivery assembly 620 and an ablation assembly 610.

The distal end of the delivery assembly 620 in this embodiment is connected to the sleeve 100 according to any of the above embodiments. The ablation assembly 610 is movably inserted in the delivery assembly 210, and the ablation assembly 610 includes an ablation needle 612. The delivery assembly 620 is configured to be introduced into the heart 1000 through lumens. After the ablation needle 612 moves out from the delivery assembly 620 and the sleeve 100, it enters the myocardial tissue by puncturing the endocardium to ablate the myocardial tissue.

Referring to FIG. 17, the delivery assembly 620 includes a guiding sheath 400 and a bendable sheath 500. The bendable sheath 500 is movably inserted into the guiding sheath 400, and the sleeve 100 is disposed at the distal end of the bendable sheath 500. The ablation needle 612 is movably inserted into the bendable sheath 500. The sleeve 100 is driven by the bendable sheath 500 to be positioned on the tissue wall 1310.

Referring to FIG. 25, the ablation assembly 610 includes an insulating sheath 611, an ablation needle 612 and a second handle 613. The proximal end of the ablation needle 612 is connected to the second handle 613. At least part of the ablation needle 612 is provided with an ablation section, and the ablation section applied with ablation energy generated by an ablation energy generating device can perform ablation on the myocardial tissue. The insulating sheath 611 is movably arranged around the ablation needle 612 and is detachably and rotatably connected to the second handle 613. The distal end of the ablation needle 612 extends out of the insulating sheath 611, and the section of the ablation needle 612 extending out of the insulating sheath 611 performs ablation.

The following describes the ablation process of the ablation device 600 in this embodiment.

In step S21, a standard puncture via the femoral artery is performed, and the guiding sheath 400 is introduced into the human body via the femoral artery puncture and reaches the left ventricle 1300 of the heart 1000 through the aortic arch 1100, as shown in FIG. 16.

In step S22, the sleeve 100 is pushed by the bendable sheath 500 into the human body along the interior lumen of the guiding sheath 400 and reaches the left ventricle 1300, as shown in FIG. 17.

In step S23, the catheter 211 is electrically coupled to the mapping system, after the mapping system completes modeling, the bendable sheath 500 is bent and moved, the location of the moving sleeve 100 is observed from the three-dimensional image transmitted from the sleeve 100, the location is selected with the assistance of the guiding sheath 400 and the bendable sheath 500, the abutment surface 111 of the sleeve 100 is adjusted to vertically point to the tissue wall 1310 of the lesion tissue, as shown in FIG. 18.

In step S24, under the guidance of DSA (Digital subtraction angiography) and ultrasound images, the abutment surface 111 of the sleeve 100 is adjusted to abut against the tissue wall 1310 of the lesion tissue, as shown in FIG. 19.

In step S25, a contrast medium is injected into the catheter 211 so that the contrast medium flows out of the sleeve 100. Now, the DSA image is used to determine whether the abutment surface 111 is in close contact with the tissue wall 1310. If the contrast medium flows out from the first channel 120 and the second channel 130 at the same time, as shown in FIG. 21, it means that the abutment surface 111 of the sleeve 100 is still not in contact with or only in partial contact with the tissue wall 1310 of the lesion tissue. FIG. 22 shows that the abutment surface 111 of the sleeve 100 is only in partial contact with the tissue wall 1310 of the lesion tissue. If the contrast medium only flows out from the second channel 130 of the sleeve 100, as shown in FIG. 20, it means that the abutment surface 111 of the sleeve 100 has closely contacted the tissue wall 1310, and the next step can be performed. Otherwise, steps S13 and S14 need to be repeated until the abutment surface 111 of the sleeve 100 is in close contact with the tissue wall 1310.

In step S26, as shown in FIGS. 23 and 24, the ablation needle 612 is driven by the second handle 613 to penetrate into the tissue wall 1310, and the ablation energy generating device provides ablation energy to the ablation needle 612 to ablate the lesion tissue.

In step S27, the above steps are repeated to find the next ablation point. After ablations at all desired ablation points, the guiding sheath 400, the bendable sheath 500, the sleeve 100 and the ablation needle 612 are withdrawn, thereby finishing the ablation.

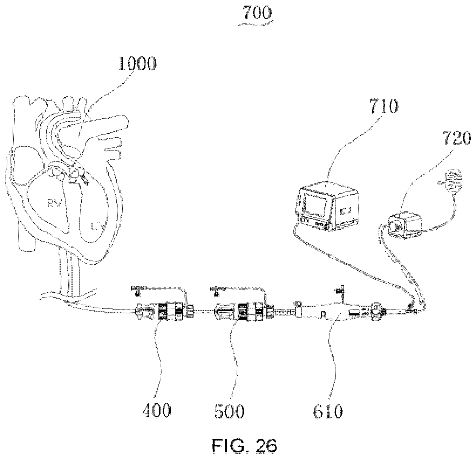

Referring to FIG. 26, in a sixth embodiment, the present disclosure further provides an ablation system 700, which includes an ablation energy generating device 710 and the above-mentioned ablation device 600.

The ablation energy generating device 710 is coupled with the ablation device 600 to provide energy for the ablation device 600.

It can be understood that the temperature of the tissue and blood will increase during the ablation process. In order to protect the human body, the ablation system 700 further includes a perfusion device 720. The perfusion device 720 is used to provide liquid to the ablation device 600, so that the perfusion device 720 can provide liquid to the periphery of the tissue wall 1310, to enlarge the ablation area and cool the tissue and blood within the ablation area. The fluid provided by the perfusion device 720 can be cold saline.

The technical solutions of the present disclosure are not limited to the technical solutions disclosed in the above embodiments, but further include technical solutions by combining the above technical features in any suitable manner. It should be noted that those skilled in the art can make several developments and modifications without departing from the principles of the present disclosure, which also fall into the protection scope of the present disclosure.

## Claims

1. A sleeve, comprising:
a main body, wherein the main body defines a first channel passing through the main body in an axial direction, and a side wall of the main body defines at least one second channel which is communicated with the first channel.

2. The sleeve of claim 1, wherein the main body comprises a distal section, and the distal section is provided with an abutment surface for abutting a myocardial tissue.

3. The sleeve of claim 2, wherein the sleeve further comprises at least one sliding member, at least a part of the sliding member protrudes from the abutment surface, and the sliding member is configured to selectively open or close the second channel.

4. The sleeve of claim 3, wherein the sliding member has a first position for opening the second channel and a second position for closing the second channel, and the sleeve further comprises at least one spring element acting on the sliding member to move the sliding member toward the second position.

5. The sleeve of claim 4, wherein the sleeve further comprises at least one third channel communicated with the second channel, and the sliding member and the spring element are arranged in the third channel.

6. The sleeve of claim 5, wherein a distal end surface of the main body is the abutment surface, a distal end of the third channel defines an opening on the abutment surface, and a proximal end of the third channel is closed or provided with a blocking member, wherein a distal end of the sliding member protrudes from the abutment surface through the opening, and the sliding member is configured to selectively open or close the second channel according to a position of the sliding member in the third channel.

7. The sleeve of claim 5, wherein a proximal end of the sliding member is connected to the spring element, and a proximal end of the spring element abuts a proximal end of the third channel.

8. The sleeve of claim 5, wherein the sliding member defines a through hole, and when the spring element is in a first state, the sliding member is in the first position, and the through hole communicates with the second channel; and
when the spring element is in a second state, the sliding member is in the second position, and the sliding member blocks the second channel.

9. The sleeve of claim 5, wherein an outer wall of the main body has a balance opening communicated to a proximal end of the third channel.

10. The sleeve of claim 2, wherein the main body comprises a proximal section, an outer diameter of the proximal section gradually increases from a proximal end to a distal end, and a radial dimension of a part of the first channel corresponding to the proximal section is a constant value or gradually decreases.

11. The sleeve of claim 10, wherein the main body further comprises a middle section extending from the proximal section to the distal section, the middle section defines the second channel, and a radial dimension of a part of the middle section adjacent to the proximal section is greater than a radial dimension of a part of the middle section adjacent to the distal section.

12. The sleeve of claim 11, wherein a radial dimension of the distal section is a constant value, or a radial dimension of a part of the distal section adjacent to the middle section is greater than a radial dimension of a part of the distal section away from the middle section.

13. The sleeve of claim 1, wherein at least one electrode is provided on a side wall of the main body, or the main body is made of metal.

14. An injection device, comprising:
a delivery assembly, comprising a catheter and the sleeve of any one of claims 1 to 13, a proximal end of the sleeve being connected to a distal end of the catheter; and
an injection assembly, which is movably provided in the catheter.

15. The injection device of claim 14, wherein the injection assembly comprises a needle body, a needle connector and a needle connection tube, the needle connector is connected between the needle body and the needle connection tube, and the needle connection tube comprises an inner tube and an outer tube;
a proximal end of the needle connector is arranged around the inner tube, the outer tube is arranged around a distal end of the needle connector, and the distal end of the needle connector is arranged around the needle body;
a side wall of the needle connector defines a communication hole, and a gap between the inner tube and the outer tube is communicated to an interior of the inner tube through the communication hole.

16. The injection device of claim 14, wherein at least one electrode is provided on a side wall of the main body, or the main body is made of metal;
a conductive wire is arranged in a wall of the catheter, a distal end of the conductive wire is connected to the electrode or the main body, and a proximal end of the conductive wire is connected to a mapping system.

17. An injection system, comprising a handle and the injection device of any one of claims 14 to 16;
wherein the handle is connected to a proximal end of the catheter, the handle is provided with at least one injection part, and the injection part, the catheter, and the injection assembly are communicated with each other.

18. The injection system of claim 17, wherein the injection part comprises a first injection part and a second injection part, and the first injection part and the second injection part correspond to different injection channels in the catheter and/or the handle.

19. An ablation device, comprising a delivery assembly and an ablation assembly, a distal end of the delivery assembly being provided with the sleeve of any one of claims 1 to 13;
wherein the ablation assembly is movably inserted in the delivery assembly and comprises an ablation needle, the delivery assembly is configured to be introduced into a heart through a catheter, and the ablation needle is configured to pass through the sleeve from the delivery assembly to puncture an endocardium and enter a myocardial tissue to ablate the myocardial tissue.

20. The ablation device of claim 19, wherein the delivery assembly comprises a guiding sheath and a bendable sheath, the bendable sheath is movably inserted in the guiding sheath, the ablation needle is movably inserted in the bendable sheath, and the sleeve is disposed at a distal end of the bendable sheath.

21. The ablation device of claim 19, wherein at least part of the ablation needle is provided with an ablation section.

22. An ablation system, comprising:
the ablation device of any one of claims 19 to 21; and
an ablation energy generating device connected to the ablation device and configured to provide energy to the ablation device.

23. The ablation system of claim 22, wherein:
the ablation system further comprises a perfusion device for providing fluid to the ablation device.
